# EUROPÄISCHE PATENTANMELDUNG

(11) **EP 3 680 014 A1**
(43) Veröffentlichungstag der Anmeldung: **15.07.2020**
(21) Anmeldenummer: 20150484.2
(22) Anmeldetag: 07.01.2020
(51) Int. Cl.: B01J 23/44, C07C 319/18, B01J 21/04, B01J 35/00, B01J 35/10, B01J 37/02

(54) **KATALYSATOR UND VERFAHREN ZUR ENTFERNUNG VON MERCAPTANEN AUS KOHLENWASSERSTOFFSTRÖMEN**

(30) Priorität: 08.01.2019 EP 19150660
(71) Anmelder: Evonik Operations GmbH, 45128 Essen (DE)
(72) Erfinder: Peitz, Stephan, 45739 Oer-Erkenschwick (DE); Reeker, Helene, 44227 Dortmund (DE); Bukohl, Reiner, 45770 Marl (DE); Quandt, Thomas, 45772 Marl (DE); Röder, Stefan, 36391 Sinntal (DE); Rix, Armin Matthias, 45770 Marl (DE); Wolff, Andreas, 45657 Recklinghausen (DE); Stochniol, Guido, 45721 Haltern am See (DE)
(74) Vertreter: Evonik Patent Association

(57) **Zusammenfassung**

Die Erfindung betrifft einen Katalysator für ein Verfahren zur Entfernung von Mercaptanen und optional Disulfiden (sofern vorhanden) aus Kohlenwasserstoffströmen, insbesondere C4-Strömen, in Gegenwart höherer Diene, insbesondere C5-Diene. Gleichzeitig betrifft die Erfindung auch ein Verfahren zur Entfernung von Mercaptanen und Disulfiden (sofern vorhanden) aus Kohlenwasserstoffströmen, insbesondere C4-Strömen, in einer Ausführungsform in Gegenwart von 1-Buten, durch Thioveretherung der Mercaptane mit mehrfach ungesättigten Kohlenwasserstoffen, wobei das Verfahren in einem Reaktor unter Zusatz von Wasserstoff in Gegenwart höhere Diene, insbesondere C₅-Diene durchgeführt wird.

## Beschreibung

Die Erfindung betrifft einen Katalysator für ein Verfahren zur Entfernung von Mercaptanen und optional Disulfiden aus Kohlenwasserstoffströmen, insbesondere C4-Strömen, in Gegenwart höhere Diene, insbesondere C5-Diene. Gleichzeitig betrifft die Erfindung auch ein Verfahren zur Entfernung von Mercaptanen aus Kohlenwasserstoffströmen, insbesondere C4-Strömen, in einer Ausführungsform in Gegenwart von 1-Buten, durch Thioveretherung der Mercaptane mit mehrfach ungesättigten Kohlenwasserstoffen, wobei das Verfahren in einem Reaktor unter Zusatz von Wasserstoff in Gegenwart höhere Diene, insbesondere C₅-Diene durchgeführt wird.

Gemische aus C4-Kohlenwasserstoffen sind Rohstoffe der Petro-Folgechemie. Sie stammen entweder aus Steamcrackern (so genanntes "Crack-C4") oder aus fluid-katalytischen Crackern (so genanntes "FCC-C4"). Es werden auch Gemische von C4-Kohlenwasserstoffen unterschiedlicher Herkunft gehandelt, so genannter "C4-Schnitt". Zum Zwecke der Verwertung der einzelnen Komponenten sind die C4-Gemische möglichst sortenrein in ihre Bestandteile zu zerlegen.

Mercaptane sind Verbindungen der Klasse R-SH, wobei R für einen Kohlenwasserstoffrest steht und S für Schwefel und H für Wasserstoff. Mercaptane werden auch als Thiole bezeichnet. Wichtige Vertreter der Mercaptane sind Methylmercaptan und Ethylmercaptan, auch Methanthiol bzw. Ethanthiol genannt. Disulfide sind im Sinne der vorliegenden Erfindung organische Verbindungen, die eine S-S-Bindung aufweisen. Ein wichtiger Vertreter ist das Dimethyldisulfid. Mercaptane treten mit bis zu 200 ppm als unerwünschte Begleitstoffe in C4-Kohlenwasserstoffgemischen auf.

Technische C4-Kohlenwasserstoffgemische aus katalytischen Crackern (FCC-C4) oder Steamcrackern (Crack-C4) enthalten üblicherweise neben gesättigten und einfach ungesättigten Verbindungen auch mehrfach ungesättigte Verbindungen. Bevor einzelne Verbindungen aus diesen Gemischen isoliert werden können, ist es häufig notwendig, andere Verbindungen möglichst vollständig zu entfernen. Dies kann durch physikalische Methoden, wie z. B. Destillation, Extraktivdestillation oder Extraktion, aber auch durch eine selektive chemische Umsetzung der zu entfernenden Komponenten erfolgen. Besonderes Augenmerk muss dabei auf der möglichst vollständigen Entfernung von den im C4-Kohlenwasserstoffgemisch enthaltenden Verunreinigungen, wie Sauerstoff-, Stickstoff- und Schwefelhaltigen Komponenten liegen, da diese als Katalysatorgifte negative Auswirkungen auf die einzelnen Prozessschritte haben können. Während diese Verunreinigung typischerweise in Crack C4 nur in Spuren vorhanden sind, können diese in FCC C4-Strömen auch in höheren Konzentrationen vorhanden sein.

Die Zusammensetzung der Rohstoffe kann je nach Herkunft des Materials stark schwanken. Zu den aufgeführten C4-Komponenten gesellen sich noch Kohlenwasserstoffe mit weniger oder mehr Kohlenstoffatomen, sowie Verunreinigungen wie Mercaptane, Sulfide, Disulfide, Stickstoff- und sauerstoffhaltige Verbindungen in geringen Mengen.

Die Aufarbeitung von FCC-C4 kann in einer Variante so erfolgen, dass zunächst die Konzentration des Isobutans mittels eines destillativen Schrittes gesenkt wird. Gleichzeitig werden die im Gemisch vorhandenen Leichtsieder (z. B. C3-Kohlenwasserstoffe, leichte Sauerstoff, Stickstoffund Schwefelhaltige Verbindungen) entfernt bzw. minimiert. Im darauf folgenden Schritt werden in einer Kolonne alle Hochsieder (z.B. C5-Kohlenwasserstoffe, schwere Sauerstoff-, Stickstoff- und Schwefelhaltige Verbindungen) über den Sumpf entfernt. Im nächsten Schritt wird Isobuten entfernt, z. B. indem es mit Methanol zu Methyl-tert.-butylether (MTBE) umgesetzt und dieser durch Destillation entfernt wird. Soll reines Isobuten gewonnen werden, kann der Methyl-tert.-butylether anschließend wieder zu Isobuten und Methanol gespalten werden.

Zur weiteren Aufarbeitung des C4-Gemisches müssen die noch verbliebenen mehrfach ungesättigten Verbindungen mit Hilfe eines Selektivhydrierprozesses zu den entsprechenden einfach ungesättigten Verbindungen umgesetzt werden. Jetzt können 1-Buten und verbliebenes Isobutan in ausreichender Reinheit destillativ abgetrennt und die verbleibenden 2-Butene und das n-Butan weiter aufgearbeitet werden. Häufig werden die 2-Butene durch Dimerisierung zu Oktenen umgesetzt, die anschließend mittels Hydroformylierung und Hydrierung zu Weichmacheralkoholen umgesetzt werden. Die gesättigten C4-Kohlenwasserstoffe können beispielsweise als Treibmittel verwendet werden.

Wird im Selektivhydrierprozess vor der 1-Buten-Abtrennung die Konzentration der mehrfach ungesättigten Verbindung nicht auf einen Wert kleiner 10 ppm abgesenkt, so werden die Reinheitsanforderungen für 1-Buten, welches in Polymerisationen eingesetzt wird, nicht erreicht. Des Weiteren unterdrücken mehrfach ungesättigte Verbindungen die katalytische Aktivität der Katalysatoren für die Dimerisierung der 2-Butene.

Die Ansprüche an die Selektivitäten bei den Prozessen zur selektiven Hydrierung von mehrfach ungesättigten Kohlenwasserstoffen sind besonders hoch, da bei der Überhydrierung, d.h. der Hydrierung von einfach ungesättigten Verbindungen, sowie der Isomerisierung von endständigen Doppelbindungen zu innenständigen Doppelbindungen Wertprodukte vernichtet werden. Gleichzeitig müssen bei der Feinreinigung von Strömen, die bereits einen niedrigen Gehalt an mehrfach ungesättigten Verbindungen aufweisen, die Konzentrationen an mehrfach ungesättigten Verbindungen weiter auf Werte unter 10 Gew.-ppm abgesenkt werden.

Bei katalytischen C4-Strömen kann es nun vorkommen, dass neben den bereits in der Leichtsiederabtrennung destillativ entfernten leichten Schwefelhaltigen Komponenten, wie z. B. H₂S, COS oder MeSH, und den später in der C5-Kolonne abgetrennten höhersiedenden SchwefelVerbindungen, wie z. B. Dimethyldisulfid, weiterhin Mercaptan-artige Mittelsieder (z. B. Ethanthiol) enthalten sind. Diese sind nicht ohne weiteres destillativ aus dem C₄-Strom zu entfernen. Die Anwesenheit von Mercaptanen ist bei der Aufarbeitung von C4-Strömen nicht erwünscht bzw. stört diese.

Sind Mercaptane (z. B. Ethanthiol) und optional Disulfide im Zulauf zur Selektivhydrierung vorhanden, so hemmen diese die katalytische Umsetzung des 1,3-Butadiens. So können sich die verzweigten mehrfach ungesättigten Verbindungen im nachfolgenden Produkt (z. B. 1-Buten) befinden und dessen Reinheit gefährden. Gelangen die mehrfach ungesättigten Verbindungen aufgrund der mangelnden Umsetzung in der Selektivhydrierung, verursacht durch die Mercaptangehalt, in den Zulauf zur Oligomerisierung der n-Butene, so desaktivieren sie den Oligomerisierungskatalysator.

Es ist bekannt, dass sich durch Beschickung des Selektivhydrierkatalysators mit Schwefelhaltigen Komponenten hydroisomerisierungsaktive Katalysatoren bilden können. Durch die im Zulauf zur Selektivhydrierung vorhandenen Mercaptane kann ein solcher Hydroisomerisierungskatalysator gebildet werden, der zu einer unerwünschten Isomerisierung des 1-Butens zu 2-Butenen führt.

Die DE 10 2012 212 317 A1 schlägt deshalb ein Verfahren zur Thioveretherung der Mercaptane mit mehrfach ungesättigten Kohlenwasserstoffen unter Einsatz eines heterogenen Katalysators mit Palladium als katalytisch wirksamen Metall und unter Zusatz von Wasserstoff, welches in einem bestimmten Verhältnis zu den mehrfach ungesättigten Kohlenwasserstoffen eingesetzt wird, vor. Eine ausführliche Beschreibung des weiteren Standes der Technik im Hinblick auf die Entfernung von Mercaptanen aus Kohlenwasserstoffströmen findet sich ebenfalls in der DE 10 2012 212 317 A1.

Der Nachteil bei dem dort verwendeten Katalysator ist aber, dass die Thioveretherung der Mercaptane durch die Anwesenheit von höheren Dienen wie C5-Dienen, wie insbesondere Isopren, gehemmt wird und nicht mehr vollständig abläuft. Dadurch verbleiben Mercaptane, wie Ethanthiol und/oder Methanthiol im Kohlenwasserstoffstrom und können zu den bereits beschriebenen Problemen führen.

Des Weiteren werden möglicherweise im C4-Strom enthaltene Disulfide wie Dimethyldisulfid zu zwei Äquivalenten Methanthiol gespalten, wenn gleichzeitig Isopren und/oder weitere langkettige, mehrfach ungesättigte Kohlenwasserstoffe anwesend sind. Demnach erhöht sich der Anteil an Mercaptanen sogar, anstatt sich durch eine Thioveretherung zu verringern.

Die Aufgabe der vorliegenden Erfindung bestand deshalb darin, einen Katalysator bereitzustellen, der auch in Anwesenheit von höheren Dienen zu einem (nahezu) vollständigen Umsatz der im Kohlenwasserstoffstrom befindlichen Mercaptane und optional Disulfide (sofern vorhanden) führt, d.h. auch jener Mercaptane, die möglicherweise durch Disulfidspaltung zusätzlich in situ entstehen. Eine weitere Aufgabe war die Bereitstellung eines Verfahrens, welches die vorgenannten Probleme nicht aufweist und mit dem insbesondere die Thioveretherung der Mercaptane vollständig ablaufen kann.

Die Aufgabe wird durch den erfindungsgemäßen Katalysator gemäß Anspruch 1 und das Verfahren gemäß Anspruch 6 gelöst. Bevorzugte Ausführungsformen sind in den Unteransprüchen angegeben.

Gegenstand der vorliegenden Erfindung ist ein heterogener Katalysator zur Entfernung von Mercaptanen und optional Disulfiden (sofern vorhanden) aus Kohlenwasserstoffströmen in Gegenwart von höheren Dienen, wobei es sich bei dem Katalysator um einen Schalenkatalysator handelt, wobei der Katalysator Aluminiumoxid, Kieselgel oder Aktivkohle als Trägermaterial und Palladium und Platin als katalytisch wirksame Metalle umfasst und wobei der Katalysator eine Palladium-Konzentration von bis zu 2,0 Gew.-%, vorzugsweise von bis zu 1,0 Gew.-%, besonders bevorzugt von bis zu 0,5 Gew.-%, eine Platin-Konzentration von bis zu 1,0 Gew.-%, vorzugsweise von bis zu 0,5 Gew.-%, bevorzugt von bis zu 0,2 Gew.-% und eine Gesamt-Metall-Dispersion von mindestens 70% aufweist. Die Angabe der Werte für die Palladium- und die Platin-Konzentration bezieht sich jeweils auf den gesamten Katalysator. Der Begriff Schalenkatalysator meint die Form des Katalysators, bei der der Kern aus dem Trägermaterial besteht, auf dem mittels Sprühverfahren und anschließender Calcination eine Schale aus Pd/PdO und Pt/PtO aufgebracht wird, deren Schichtdicke und Eindringtiefe in den Träger jeweils nur wenige µm beträgt.

Der Kohlenwasserstoffstrom umfasst vorzugsweise C2- bis C8-Olefine, bevorzugt C3- bis C6-Olefine und ganz besonders bevorzugt C4-Olefine. Der von Mercaptanen und Disulfiden (sofern vorhanden) zu reinigende Kohlenwasserstoffstrom ist insbesondere ein Strom aus C4-Kohlenwasserstoffen (C4-Strom). Geeignete Olefine sind unter anderem α-Olefine, n-Olefine und Cycloalkene, vorzugsweise n-Olefine. In einer bevorzugten Ausführungsform handelt es sich bei dem Olefin um n-Buten. Die Olefine werden üblicherweise nicht in reiner Form eingesetzt, sondern in technisch verfügbaren Mischungen, wie beispielsweise die erwähnten Crack- oder FCC-C4. Der in dieser Erfindung zusätzlich verwendete Begriff Kohlenwasserstoffstrom ist deshalb so zu verstehend, dass er jegliche Art von Gemischen betrifft, die die entsprechenden Olefine in einer Menge enthalten, die es ermöglicht, die möglichen der Reinigung nachgeschalteten Verfahrensschritte wirtschaftlich durchzuführen.

Der Begriff "höheres Dien" meint im Sinne der vorliegenden Erfindung mindestens C5-Diene, oder Diene mit mehr als 5 Kohlenstoffatomen. Das höhere Dien ist vorzugsweise ein C5-Dien, insbesondere Isopren. Die Diene sind typischerweise in geringen Konzentrationen in den technischen Kohlenwasserstoffströmen, die gemäß der vorliegenden Erfindung von Mercaptanen zu reinigen sind, enthalten. Höhere Diene sind in der Selektivhydrierung schwieriger umzusetzen als beispielsweise Butadien (vgl. auch WO 2012/004081 A1), so dass auch in der Thioveretherung eine Hemmung der Reaktion durch C5-Diene plausibel ist.

Der Katalysator, zum Einsatz im Verfahren der vorliegenden Erfindung eingesetzt wird, ist ein Schalenkatalysator aus einem Trägermaterial und Palladium und Platin als katalytisch wirksame Metalle, wodurch die Mercaptane im Kohlenwasserstoffstrom vollständig zu Thioethern umgesetzt werden können. Bei dem Trägermaterial handelt es sich beispielsweise um Aluminiumoxid, Kieselgel oder Aktivkohle. Bevorzugt wird Aluminiumoxid als Trägermaterial eingesetzt.

Der Katalysator weist dabei eine Palladium-Konzentration von bis zu 2,0 Gew.-%, bevorzugt von bis zu 1,0 Gew.-% und besonders bevorzugt von bis zu 0,5 Gew.-% auf. Gleichzeitig weist der Katalysator eine Platin-Konzentration von bis zu 1,0 Gew.-%, bevorzugt von bis zu 0,5 Gew.-% und besonders bevorzugt von 0,2 Gew.-% auf.

Die innere Oberfläche des Katalysators beträgt vorzugsweise (bestimmt durch Gasadsorption nach DIN ISO 9277) von 50 bis 400 m²/g, weiterhin bevorzugt zwischen 100 und 300 m²/g und besonders bevorzugt zwischen 200 und 300 m²/g.

In einer bevorzugten Ausführungsform weist der Katalysator zusätzlich eine Gesamt-Metall-Oberfläche (bestimmt durch CO-Puls-Chemisorption nach AN-SOP 1954 Version 1) von mindestens 0,5 m²/g, bevorzugt von mindestens 1,0 m²/g und besonders bevorzugt von mindestens 1,5 m²/g auf. Kleinere als die angegebenen Gesamt-Metall-Oberflächen führen zu einer Reduktion der Aktivität des Katalysators.

Der erfindungsgemäße Katalysator weist eine Gesamt-Metall-Dispersion von mindestens 70%, vorzugsweise von mindestens 80% auf. Die Gesamt-Metall-Dispersion gibt an, wie gut die Metalle/Metalloxide auf der Oberfläche verteilt sind. Die Verteilung auf der Oberfläche kann über die Dauer und die Intensität des Besprühens des Trägermaterials mit der Pd/Pt-Salzlösung bei der Herstellung des Katalysators kontrolliert werden. Dispersion ist hier die Bezeichnung für ein heterogenes Gemisch aus disperser Phase (Pd/Pt) und Dispersionsmedium (Träger). Je höher die Dispersion auf der Oberfläche ist, desto höher ist die Aktivität des Katalysators. Es wurde überraschend gefunden, dass, wenn die Gesamt-Metall-Dispersion unterhalb von 70% liegt, kein Vollumsatz der Mercaptane erreicht werden kann.

Gegenstand der vorliegenden Erfindung ist weiterhin ein Verfahren zur Entfernung von Mercaptanen und Disulfiden (sofern vorhanden) aus Kohlenwasserstoffströmen in Gegenwart von mindestens einem höheren Dien durch Thioveretherung der Mercaptane mit mehrfach ungesättigten Kohlenwasserstoffen, wobei das Verfahren in einem Reaktor unter Zusatz von Wasserstoff durchgeführt wird, wobei das molare Verhältnis von Wasserstoff zu mehrfach ungesättigten Kohlenwasserstoffen maximal eins beträgt. In dem Verfahren wird der vorherig beschriebene heterogene Katalysator eingesetzt, der Palladium und Platin als katalytisch wirksame Metalle umfasst, wobei der Katalysator eine Palladium-Konzentration von bis zu 2,0 Gew.-%, vorzugsweise von bis zu 1,0 Gew.-%, besonders bevorzugt von bis zu 0,5 Gew.-%, eine Platin-Konzentration von bis zu 1,0 Gew.-%, vorzugsweise von bis zu 0,5 Gew.-%, bevorzugt von bis zu 0,2 Gew.-% und eine Gesamt-Metall-Dispersion von mindestens 70%, vorzugsweise von mindestens 80% aufweist. Die Angabe der Werte für die Palladium- und die Platin-Konzentration bezieht sich jeweils auf den gesamten Katalysator.

Der Kohlenwasserstoffstrom umfasst vorzugsweise C2- bis C8-Olefine, bevorzugt C3- bis C5-Olefine und ganz besonders bevorzugt C4-Olefine. Der von Mercaptanen zu reinigende Kohlenwasserstoffstrom ist insbesondere ein Strom aus C4-Kohlenwasserstoffen (C4-Strom). Geeignete Olefine sind unter anderem α-Olefine, n-Olefine und Cycloalkene, vorzugsweise n-Olefine. In einer bevorzugten Ausführungsform handelt es sich bei dem Olefin um n-Buten. Die Olefine werden üblicherweise nicht in reiner Form eingesetzt, sondern in technisch verfügbaren Mischungen, wie beispielsweise die erwähnten Crack- oder FCC-C4. Der in dieser Erfindung zusätzlich verwendete Begriff Kohlenwasserstoffstrom ist deshalb so zu verstehend, dass er jegliche Art von Gemischen betrifft, die die entsprechenden Olefine in einer Menge enthalten, die es ermöglicht, die möglichen der Reinigung nachgeschalteten Verfahrensschritte wirtschaftlich durchzuführen.

Der Begriff "höheres Dien" meint im Sinne der vorliegenden Erfindung mindestens C5-Diene, oder Diene mit mehr als 5 Kohlenstoffatomen. Das höhere Dien ist vorzugsweise ein C5-Dien, insbesondere Isopren. Die Diene sind typischerweise in geringen Konzentrationen in den technischen Kohlenwasserstoffströmen, die gemäß der vorliegenden Erfindung von Mercaptanen zu reinigen sind, enthalten. Höhere Diene sind in der Selektivhydrierung schwieriger umzusetzen als Butadien (vgl. auch WO 2012/004081 A1), so dass auch in der Thioveretherung eine Hemmung der Reaktion durch C5-Diene plausibel ist.

Das erfindungsgemäße Verfahren ist durch Einsatz eines heterogenen Katalysators, der Palladium und Platin als katalytisch wirksame Metalle umfasst, in der Lage, Mercaptane unter vollständigem Umsatz in hochsiedende Thioether umzusetzen, gleichzeitig eine signifikante Isomerisierung von 1-Buten zu innenständigen Butenen nahezu vollständig zu unterdrücken, sowie eine Hydrierung der Butene vollständig zu verhindern. Für den Fall, dass 1-Buten im Kohlenwasserstoffstrom in einer Konzentration enthalten ist, die höher ist als es dem thermodynamischen Gleichgewicht der Doppelbindungsisomerisierung von 1-Buten zu 2-Buten entspricht, so ist der Umsatz von 1-Buten in der Isomerisierung zu 2-Butenen und/oder der Hydrierung zu n-Butan vorzugsweise kleiner als 5%, besonders bevorzugt kleiner als 3% und ganz besonders bevorzugt kleiner als 2%.

Es hat sich gezeigt, dass Mercaptane in Gegenwart von mehrfach ungesättigten Kohlenwasserstoffen und Wasserstoff bis unter die Nachweisgrenze zu höhersiedenden Thioethern umgesetzt werden können, wobei die Isomerisierung von 1-Buten stark zurückgedrängt und die Hydrierung von Butenen komplett verhindert wird. Die Nachweisgrenze der Mercaptane liegt derzeit bei etwa 50 ppbw, also bei einem Gewichtsanteil von 50 *10⁻⁹.

Im Rahmen der vorliegenden Erfindung ist die einzuhaltende Wasserstoffmenge, die im Verfahren eingesetzt wird, maximal eins (äquimolar) zu den im Kohlenwasserstoffgemisch enthaltenden mehrfach ungesättigten Kohlenwasserstoffen. Bevorzugt liegt das molare Verhältnis von Wasserstoff zu den mehrfach ungesättigten Kohlenwasserstoffen zwischen 0,01 und 0,8. Besonders bevorzugt liegt es zwischen 0,1 und 0,5.

Ein großer Vorteil dieses Verfahrens ist, das aufgrund des geringen Wasserstoffanteils im C4-Strom enthaltendes 1-Buten kaum isomerisiert wird und weiterhin als Wertprodukt zur Verfügung steht. Nur durch die genau einzuhaltenden Grenzen für die zugeführte Wasserstoffmenge wird erreicht, dass Mercaptane in einem Verfahren auf Konzentrationswerte von unter 50 ppbw mit mehrfach ungesättigten C4-Kohlenwasserstoffen zu hochsiedenden Thioethern verethert werden können, ohne dass dabei eine Hydrierung der ebenfalls im Zulauf enthaltenen einfach ungesättigten Butene sowie eine nennenswerte Isomerisierung des 1-Butens erfolgt. Auf Wasserstoff kann jedoch nicht gänzlich verzichtet werden, da ohne Wasserstoff kein Umsatz an Mercaptanen erfolgt.

Ein weiterer Vorteil des erfindungsgemäßen Verfahrens ist, dass es auch in Gegenwart von höheren Dienen zum einem vollständigen Umsatz von Mercaptanen führt.

Bei den mehrfach ungesättigten Kohlenwasserstoffen, welche mit den Mercaptanen thioverethert werden, handelt es sich vorzugsweise um 1,3-Butadien, und/oder um But-3-en-1-in und/oder um 1,2-Butadien. Diese Diene und Acetylene sind insbesondere in FCC-C4 nur in geringen Mengen vorhanden und müssen stromabwärts ohnehin vollständig hydriert werden und stehen damit nicht mehr als Wertprodukt zur Verfügung. Aus stark 1,3-Butadien-halten Crack C4-Strömen wird das 1,3 Butadien zuvor gesondert entfernt und verwertet. Die dabei im C4-Strom verbleibenden restlichen Butadiene können dann für die Thioveretherung genutzt werden.

Ein weiterer besonderer Vorteil des Verfahrens ist, dass neben dem hochreaktiven Mercaptan Methanthiol auch höhere Mercaptane (z.B. Ethanthiol) umgesetzt und dadurch entfernt werden können.

Dem Kohlenwasserstoffgemisch kann im Verfahren optional zusätzlich Kohlenmonoxid zugegeben werden. Der Gehalt an Kohlenmonoxid im Zulauf beträgt in diesem Fall zwischen 0.05 und 20 ppm Kohlenmonoxid, bezogen auf die Masse des Kohlenwasserstoffgemisches. Bevorzugt wird zwischen 0.5 und 5 ppm Kohlenmonoxid zugegeben. Dosierungen oberhalb von 20 ppm verbessern die Ergebnisse nicht mehr. Das Kohlenmonoxid wird separat in den Reaktor eindosiert oder dem einlaufenden C4-Strom mitgegeben. Kohlenmonoxid kann als zusätzlicher Moderator wirken, der eine Isomerisierung von 1-Buten zu 2-Butenen reduziert.

Die Eintrittstemperatur des Reaktorzulaufs liegt vorzugsweise im Bereich von 0 bis 180°C, bevorzugt im Bereich von 60 bis 150°C, besonders bevorzugt im Bereich von 80 bis 130°C. Der Druck liegt vorzugsweise im Bereich von 2 bis 50 bar, bevorzugt im Bereich von 6 bis 40 bar, besonders bevorzugt im Bereich von 10 bis 30 bar. In einer bevorzugten Ausführungsform liegt der Wasserstoff im Kohlenwasserstoffstrom vollständig gelöst vor. In diesem Fall muss der Druck so gewählt werden, dass der Wasserstoff vollständig gelöst bleibt und im Reaktor keine Gasphase auftritt.

Die Thioveretherung wird vorzugsweise als Flüssigphasenverfahren betrieben. Dies bedeutet, dass sämtliche Komponenten am Katalysator in flüssiger Phase vorliegen oder flüssig in den Reaktor eingebracht werden. Insbesondere bedeutet das, dass der Wasserstoff und ggf. auch das Kohlenmonoxid in der flüssigen Phase vollständig gelöst sind. Das Zugeben von Wasserstoff zu dem zu hydrierenden Gemisch aus Kohlenwasserstoffen erfolgt somit in fein verteilter Form und in solchen Mengen, bei denen stets eine homogene Flüssigphase vor Eintritt in den Reaktor vorhanden ist

Die zu verethernden Kohlenwasserstoffgemische können Mercaptane und Disulfide (sofern vorhanden) Im Bereich von 0,01 und 200 Gew.-ppm enthalten. Die Thioveretherung kann in einer oder mehreren Reaktionsstufen durchgeführt werden. Falls eine so große Menge an Mercaptanen und Disulfide (sofern vorhanden) im Zulauf enthalten ist, dass die notwendige Menge Wasserstoff nicht mehr im Zulauf löslich ist, kann der Zulauf durch Kreislauffahrweise verdünnt werden. Alternativ kann der Wasserstoff in mehreren Teilmengen, verteilt über die Reaktorlänge bzw. über die einzelnen Reaktionsstufen, zugegeben werden.

Nach dem Umsatz der Mercaptane inklusive der aus den optional vorliegenden Disulfiden entstandenen Mercaptane zu hochsiedenden Thioethern ist eine destillative Abtrennung dieser Thioether möglich. Dadurch kann der Thioethergehalt des verbleibenden C4-Kohlenwasserstoffgemisches auf unter 50 Gew.-ppb verringert werden. Zusammen mit einem möglichen dem Thioveretherungsreaktor vorgeschalteten Leichtsiederabtrennung und einer dem Thioveretherungsreaktor nachgeschalteten Schwersiederdestillation ist somit eine vollständige Entfernung aller Schwefelhaltigen Komponenten aus dem C₄-Kohlenwasserstoffgemisch möglich.

Die Konzentration an mehrfach ungesättigten Olefinen kann mittels Gas-Chromatographie online gemessen und die Wasserstoffmenge exakt danach eingestellt werden. Dies gilt ebenfalls für die Schwefelverbindungen.

Das Verfahren wird bevorzugt auf Mercaptan-haltige Gemische aus C₄-Kohlenwasserstoffen angewendet, die aus katalytischen Crackern (FCC C4) oder aus Steamcrackern (Crack C4) stammen. Selbstverständlich kann auch C4-Schnitt verarbeitet werden.

Bevorzugt wird das als Zulauf verwendete C₄-Kohlenwasserstoffgemisch zuvor einer destillativen Abtrennung von Leichtsiedern, insbesondere von Isobutan unterzogen. Alternativ wird das Verfahren vor der Abtrennung von Isobutan angewendet.

Im Folgenden wird die vorliegende Erfindung durch Beispiele erläutert. Diese Beispiele sind jedoch nur exemplarisch ausgewählt und nicht einschränkend zu verstehen.

### Beispiel 1 (nicht erfindungsgemäß):

In einen Rohrreaktor werden 540 ml eines Katalysators mit 0,5 Gew.-% Pd auf Al₂O₃ eingefüllt. Der Reaktor wird mit 4 kg/h eines C4-Gemisches kontinuierlich bei 90°C und 20 bar Druck durchströmt. Das C4-Gemisch weist die folgende Zusammensetzung auf:
0,5 Gew.-% 1,3-Butadien, 13,8 Gew.-% Butane, 40,3 Gew.-% 2-Butene, 20,3 Gew.-% 1-Buten, 22,0 Gew.-% Isobuten und 3,1 Gew.-% C5-Kohlenwasserstoffe, sowie 120 Gew.-ppm Isopren, 2,8 Gew.-ppm Schwefel aus Ethanthiol und 6,0 Gew.-ppm Schwefel aus Dimethyldisulfid.

Zu diesem Gemisch werden vor dem Reaktor 4,4 Nl/h Wasserstoff dosiert. Der Austrag des Reaktors setzt sich wie folgt zusammen:
0,4 Gew.-% 1,3-Butadien, 14,1 Gew.-% Butane, 40,3 Gew.-% 2-Butene, 20,3 Gew.-% 1-Buten, 21,8 Gew.-% Isobuten und 3,1 Gew.-% C5-Kohlenwasserstoffe, sowie 100 Gew.-ppm Isopren, 0,7 Gew.-ppm Schwefel aus Ethanthiol und 2,2 Gew.-ppm Schwefel aus Dimethyldisulfid. Zusätzlich gefunden wurden 1,5 Gew.-ppm Schwefel aus Methanthiol, sowie 4,4 Gew.-ppm an Schwefel aus hochsiedenden Verbindungen.

Dies entspricht einem Umsatz von 75% Ethanthiol, sowie 38,3% Umsatz des Dimethyldisulfids zu hochsiedenden Schwefelverbindungen und 25% Umsatz des Dimethyldisulfids zu Methanthiol.

Es konnte kein Vollumsatz an Ethanthiol erreicht werden, zudem wurde mit Methanthiol ein weiteres Mercaptan aus Dimethyldisulfid gebildet.

### Beispiel 2 (erfindungsgemäß):

In einen Rohrreaktor werden 540 ml eines Katalysators mit 0,5 Gew.-% Pd und 0,2 Gew.-% Pt auf Al₂O₃ eingefüllt. Der Reaktor wird mit 4 kg/h eines C4-Gemisches kontinuierlich bei 90°C und 20 bar Druck durchströmt. Das C4-Gemisch weist die folgende Zusammensetzung auf:
0,6 Gew.-% 1,3-Butadien, 12,3 Gew.-% Butane, 40,5 Gew.-% 2-Butene, 19,5 Gew.-% 1-Buten, 23,8 Gew.-% Isobuten und 3,3 Gew.-% C5-Kohlenwasserstoffe, sowie 95 Gew.-ppm Isopren, 3,4 Gew.-ppm Schwefel aus Ethanthiol und 3,2 Gew.-ppm Schwefel aus Dimethyldisulfid.

Zu diesem Gemisch werden vor dem Reaktor 4,4 Nl/h Wasserstoff dosiert. Der Austrag des Reaktors setzt sich wie folgt zusammen:
0,5 Gew.-% 1,3-Butadien, 12,6 Gew.-% Butane, 39,5 Gew.-% 2-Butene, 19,5 Gew.-% 1-Buten, 24,6 Gew.-% Isobuten und 3,3 Gew.-% C5-Kohlenwasserstoffe, sowie 80 Gew.-ppm Isopren, 0,0 Gew.-ppm Schwefel aus Ethanthiol und 1,2 Gew.-ppm Schwefel aus Dimethyldisulfid, sowie 5,4 Gew.-ppm an Schwefel aus hochsiedenden Verbindungen.

Es konnte kein Methanthiol nachgewiesen werden. Dies entspricht einem Umsatz von 100% Ethanthiol, sowie 62,5% Umsatz des Dimethyldisulfids zu hochsiedenden Schwefelverbindungen.

Durch Verwendung eines erfindungsgemäßen Katalysators konnte nach den Beispielen 1 und 2 der Umsatz an Mercaptanen gegenüber dem nicht erfindungsgemäßen Katalysator deutlich gesteigert werden.

### Beispiel 3 (nicht erfindungsgemäß):

In einen Rohrreaktor werden 540 ml eines Katalysators mit 0,5 Gew.-% Pd und 0,2% Pt auf Al₂O₃ eingefüllt. Der Katalysator weist folgende Charakteristika auf, die mittels CO-Puls-Chemisorption nach AN-SOP 1954 Version 1 bestimmt wurden:

| Metall-Oberfläche [m²/g] | 1,3 |
|---|---|
| gesamte adsorbierte Gasmenge [mmol/g] | 20,3 |
| Gesamt-Metall-Dispersion [%] | 65 |

Der Reaktor wird mit 4 kg/h eines C4-Gemisches kontinuierlich bei 90°C und 20 bar Druck durchströmt. Das C4-Gemisch weist die folgende Zusammensetzung auf: 0,7 Gew.-% 1,3-Butadien, 13,6 Gew.-% Butane, 40,4 Gew.-% 2-Butene, 20,3 Gew.-% 1-Buten, 22,2 Gew.-% Isobuten und 2,9 Gew.-% C5-Kohlenwasserstoffe, sowie 87 Gew.-ppm Isopren, 5,1 Gew.-ppm Schwefel aus Ethanthiol und 5,1 Gew.-ppm Schwefel aus Dimethyldisulfid.

Zu diesem Gemisch werden vor dem Reaktor 5,3 Nl/h Wasserstoff dosiert. Der Austrag des Reaktors setzt sich wie folgt zusammen:
0,4 Gew.-% 1,3-Butadien, 13,7 Gew.-% Butane, 39,1 Gew.-% 2-Butene, 20,8 Gew.-% 1-Buten, 23,2 Gew.-% Isobuten und 2,9 Gew.-% C5-Kohlenwasserstoffe, sowie 72 Gew.-ppm Isopren, 1,4 Gew.-ppm Schwefel aus Ethanthiol und 1,7 Gew.-ppm Schwefel aus Dimethyldisulfid. Zusätzlich gefunden wurden 0,9 Gew.-ppm Schwefel aus Methanthiol, sowie 6,2 Gew.-ppm an Schwefel aus hochsiedenden Verbindungen.

Dies entspricht einem Umsatz von 72,5% Ethanthiol, sowie 35,3% Umsatz des Dimethyldisulfids zu hochsiedenden Schwefelverbindungen und 17,6% Umsatz des Dimethyldisulfids zu Methanthiol. Es konnte kein Vollumsatz an Ethanthiol erreicht werden, zudem wurde mit Methanthiol ein weiteres Mercaptan aus Dimethyldisulfid gebildet.

### Beispiel 4 (erfindungsgemäß):

In einen Rohrreaktor werden 540 ml eines Katalysators mit 0,5 Gew.-% Pd und 0,2 Gew.-% Pt auf Al₂O₃ eingefüllt. Der Katalysator weist folgende Charakteristika auf, die mittels CO-Puls-Chemisorption nach AN-SOP 1954 Version 1 bestimmt wurden:

| | |
|---|---|
| Metall-Oberfläche [m²/g] | 1,6 |
| gesamte adsorbierte Gasmenge [mmol/g] | 25,5 |
| Gesamt-Metall-Dispersion [%] | 81,9 |

Der Reaktor wird mit 4 kg/h eines C4-Gemisches kontinuierlich bei 90°C und 20 bar Druck durchströmt. Das C4-Gemisch weist die folgende Zusammensetzung auf:
0,7 Gew.-% 1,3-Butadien, 13,0 Gew.-% Butane, 39,8 Gew.-% 2-Butene, 19,2 Gew.-% 1-Buten, 23,9 Gew.-% Isobuten und 3,4 Gew.-% C5-Kohlenwasserstoffe, sowie 112 Gew.-ppm Isopren, 5,7 Gew.-ppm Schwefel aus Ethanthiol und 5,4 Gew.-ppm Schwefel aus Dimethyldisulfid.

Zu diesem Gemisch werden vor dem Reaktor 5,6 Nl/h Wasserstoff dosiert. Der Austrag des Reaktors setzt sich wie folgt zusammen:
0,4 Gew.-% 1,3-Butadien, 13,0 Gew.-% Butane, 40,4 Gew.-% 2-Butene, 18,9 Gew.-% 1-Buten, 23,8 Gew.-% Isobuten und 3,4 Gew.-% C5-Kohlenwasserstoffe, sowie 96 Gew.-ppm Isopren, 0,0 Gew.-ppm Schwefel aus Ethanthiol und 0,7 Gew.-ppm Schwefel aus Dimethyldisulfid.

Es konnte kein Methanthiol nachgewiesen werden. Dies entspricht einem Umsatz von 100% Ethanthiol, sowie 87% Umsatz des Dimethyldisulfids zu hochsiedenden Schwefelverbindungen.

### Beispiel 5 (erfindungsgemäß):

In einen Rohrreaktor werden 540 ml eines Katalysators mit 0,5 Gew.-% Pd und 0,2 Gew.-% Pt auf Al₂O₃ eingefüllt. Der Katalysator weist folgende Charakteristika auf, die mittels CO-Puls-Chemisorption nach AN-SOP 1954 Version 1 bestimmt wurden:

| | |
|---|---|
| Metall-Oberfläche [m²/g] | 1,5 |
| gesamte adsorbierte Gasmenge [mmol/g] | 23,8 |
| Gesamt-Metall-Dispersion [%] | 76 |

Der Reaktor wird mit 4 kg/h eines C4-Gemisches kontinuierlich bei 90°C und 20 bar Druck durchströmt. Das C4-Gemisch weist die folgende Zusammensetzung auf:
0,6 Gew.-% 1,3-Butadien, 12,4 Gew.-% Butane, 39,8 Gew.-% 2-Butene, 19,9 Gew.-% 1-Buten, 23,9 Gew.-% Isobuten und 3,8 Gew.-% C5-Kohlenwasserstoffe, sowie 124 Gew.-ppm Isopren, 3,3 Gew.-ppm Schwefel aus Ethanthiol und 2,2 Gew.-ppm Schwefel aus Dimethyldisulfid.

Zu diesem Gemisch werden vor dem Reaktor 4,4 Nl/h Wasserstoff dosiert. Der Austrag des Reaktors setzt sich wie folgt zusammen:
0,4 Gew.-% 1,3-Butadien, 12,6 Gew.-% Butane, 39,8 Gew.-% 2-Butene, 19,7 Gew.-% 1-Buten, 23,4 Gew.-% Isobuten und 3,8 Gew.-% C5-Kohlenwasserstoffe, sowie 104 Gew.-ppm Isopren, 0,0 Gew.-ppm Schwefel aus Ethanthiol und 0,6 Gew.-ppm Schwefel aus Dimethyldisulfid.

Es konnte kein Methanthiol nachgewiesen werden. Dies entspricht einem Umsatz von 100% Ethanthiol, sowie 72,7% Umsatz des Dimethyldisulfids zu hochsiedenden Schwefelverbindungen.

Im Vergleich der Beispiele 3 bis 5 zeigt sich, dass bei der Verwendung von Katalysatoren mit einer Gesamt-Metall-Dispersion von weniger als 70% kein Vollumsatz der Mercaptane erreicht werden kann. Dieser wird nach den Beispielen 4 und 5 erst dann erreicht, wenn die Gesamt-Metall-Dispersion auf dem Katalysator oberhalb von 70% liegt.

## Patentansprüche

1. Heterogener Katalysator Entfernung von Mercaptanen und optional Disulfiden aus Kohlenwasserstoffströmen in Gegenwart von höheren Dienen, wobei es sich bei dem Katalysator um einen Schalenkatalysator handelt, **dadurch gekennzeichnet, dass** der Katalysator Aluminiumoxid, Kieselgel oder Aktivkohle als Trägermaterial und Palladium und Platin als katalytisch wirksame Metalle umfasst, wobei der Katalysator eine Palladium-Konzentration von bis zu 2,0 Gew.-%, eine Platin-Konzentration von bis zu 1,0 Gew.-% und eine Gesamt-Metall-Dispersion von mindestens 70% aufweist.

2. Heterogener Katalysator nach Anspruch 1, **dadurch gekennzeichnet, dass** der Katalysator eine Palladium-Konzentration von bis zu 1,0 Gew.-%, bevorzugt von bis zu 0,5 Gew.-% enthält.

3. Heterogener Katalysator nach Anspruch 1 oder 2, **dadurch gekennzeichnet, dass** der Katalysator eine Platin-Konzentration von bis zu 0,5 Gew.-%, bevorzugt von bis zu 0,2 Gew.-% enthält.

4. Heterogener Katalysator nach einem der Ansprüche 1 bis 3, **dadurch gekennzeichnet, dass** der Katalysator eine Gesamt-Metall-Oberfläche von mindestens 0,5 m²/g, bevorzugt mindestens 1,0 m²/g, besonders bevorzugt mindestens 1,5 m²/g aufweist.

5. Heterogener Katalysator einem der Ansprüche 1 bis 4, **dadurch gekennzeichnet, dass** der Katalysator eine Gesamt-Metall-Dispersion von mindestens 80% aufweist.

6. Verfahren zur Entfernung von Mercaptanen und Disulfiden (sofern vorhanden) aus Kohlenwasserstoffströmen in Gegenwart von mindestens einem höheren Dien durch Thioveretherung der Mercaptane mit mehrfach ungesättigten Kohlenwasserstoffen, wobei das Verfahren in einem Reaktor unter Zusatz von Wasserstoff durchgeführt wird, wobei das molare Verhältnis von Wasserstoff zu mehrfach ungesättigten Kohlenwasserstoffen maximal eins beträgt, **dadurch gekennzeichnet, dass** in dem Verfahren der heterogene Katalysator eingesetzt wird, der Palladium und Platin als katalytisch wirksame Metalle umfasst, wobei der Katalysator eine Palladium-Konzentration von bis zu 2,0 Gew.-%, eine Platin-Konzentration von bis zu 1,0 Gew.-% und eine Gesamt-Metall-Dispersion von mindestens 70% aufweist.

7. Verfahren nach Anspruch 6, **dadurch gekennzeichnet, dass** das höhere Dien ein C₅-Dien ist, vorzugsweise Isopren.

8. Verfahren nach Anspruch 6 oder 7, **dadurch gekennzeichnet, dass** die Mercaptane mit mehrfach ungesättigten Kohlenwasserstoffen thioverethert werden, wobei die mehrfach ungesättigten Kohlenwasserstoffe aus der Gruppe, bestehend aus 1,3-Butadien, But-3-en-1-in und 1,2-Butadien, ausgewählt werden.

9. Verfahren nach einem der Ansprüche 6 bis 8, **dadurch gekennzeichnet, dass** als Mercaptane mindestens Ethanthiol und/oder Methanthiol vorhanden sind.

10. Verfahren nach einem der Ansprüche 6 bis 9, **dadurch gekennzeichnet, dass** das molare Verhältnis von Wasserstoff zu mehrfach ungesättigten Kohlenwasserstoffen zwischen 0,01 und 0,8 beträgt; bevorzugt, dass es zwischen 0,1 und 0,5 beträgt.

11. Verfahren nach einem der Ansprüche 6 bis 10, **dadurch gekennzeichnet, dass** es in Gegenwart von Kohlenmonoxid durchgeführt wird, wobei der Gehalt an Kohlenmonoxid im Zulauf des Reaktors weniger als 20 ppm beträgt, bezogen auf die Masse des Zulaufes.

12. Verfahren nach einem der Ansprüche 6 bis 11, **dadurch gekennzeichnet, dass** die Eintrittstemperatur des Zulaufs in den Reaktor zwischen 0°C und 180°C, vorzugsweise zwischen 60°C und 150°C und ganz besonders bevorzugt zwischen 80°C und 130°C beträgt.

13. Verfahren nach einem der Ansprüche 6 bis 12, **dadurch gekennzeichnet, dass** es als Flüssigphasenverfahren betrieben wird und der Wasserstoff in der flüssigen Phase vollständig gelöst ist.

14. Das Verfahren nach einem der Ansprüche 6 bis 13, **dadurch gekennzeichnet, dass** als Zulauf für den Reaktor ein Strom aus C₄-Kohlenwasserstoffen verwendet wird.

15. Verfahren nach einem der Ansprüche 6 bis 14, **dadurch gekennzeichnet, dass** wenn 1-Buten im Kohlenwasserstoffstrom in einer Konzentration enthalten ist, die höher liegt als es dem thermodynamischen Gleichgewicht der Doppelbindungsisomerisierung 1-Buten zu 2-Buten entspricht, der Umsatz von 1-Buten in der Isomerisierung zu 2-Butenen und/oder der Hydrierung zu n-Butan kleiner als 5%, bevorzugt kleiner als 3% und ganz besonders bevorzugt kleiner als 2% ist.
